# EUROPEAN PATENT APPLICATION

(11) **EP 3 698 841 A1**
(43) Date of publication of application: **26.08.2020**
(21) Application number: 19305199.2
(22) Date of filing: 19.02.2019
(51) Int. Cl.: A61M 37/00

(54) **INJECTION DEVICE**

(71) Applicant: L'OREAL, 75008 Paris (FR)
(72) Inventor: BORDEAUX, Dominique, 94152 CHEVILLY LARUE (FR); DONNELLY, Ryan, Belfast BT9 7BL (GB); LARRANETA LANDA, Eneko, Belfast BT9 7BL (GB); DOMINGUEZ ROBLES, Juan, Belfast BT9 7BL (GB)
(74) Representative: Nony

(57) **Abstract**

The invention relates an injection system (10) for injecting at least one material into human keratinous materials, comprising:
o At least one treatment unit (20) defining at least one cavity and at least one micro-implant (40) made of said at least one material received in said cavity of the unit and having at least one portion having a same shape as the cavity;
o an injection device (30) configured for applying a pressure to the at least one micro-implant (40) to expel the latter out of the unit (20) into the keratinous materials.

## Description

### Field of the invention

The present invention relates to devices for injecting at least one material in keratinous materials such human skin and mucous membranes.

### Background of the invention

US 8167852 and US 8834423 disclose devices that comprise an array of micro-implants (also called "microneedles") carried by a support.

Such devices may be useful for delivery of cosmetic materials, drugs or vaccine into the skin. An advantage is that they can penetrate the stratum corneum, epidermis or dermis without the pain caused by conventional needles and can be self-administered.

The production of solid microneedles arrays has been described in various applications such as WO2008/139786, WO2009/040548, WO2015/147040 and WO2016/076442.

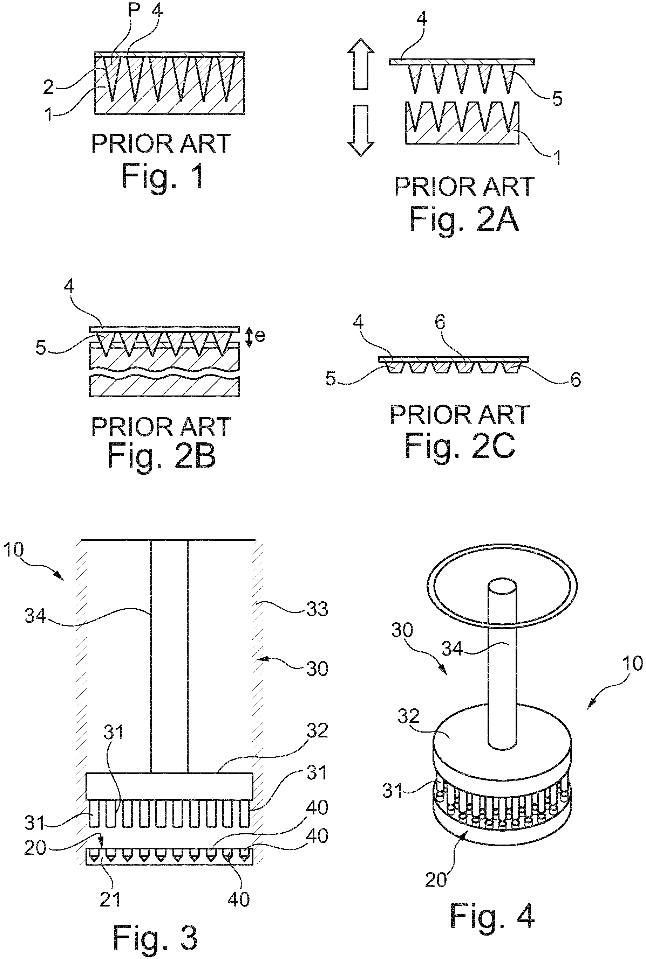

Micro-implants devices may thus be manufactured using, as shown in Fig.1, a mold 1 provided with cavities 2 having the shape to be given to the micro-implants. A product P is poured in the mold 1 and a backplate 4 is pressed against the product present at the opening of the cavities. When the micro-implants 5 are unmolded, as shown in Figure 2A, they remain attached to the backplate 4.

The micro-implants need then to be packaged in a suitable sterile packaging (not shown) until use. The packaging should protect them from mechanical damage and should be hard. This increases cost and waste.

At the time of use, the device is pressed into the skin as shown in Figure 2B, and the micro-implants penetrate in the stratum corneum, epidermis or dermis. It is difficult to press evenly the micro-implants due to suppleness of the skin and there may remain a portion of thickness e of the micro-implants that is not inserted into the stratum corneum, epidermis or dermis. When the backplate 4 is removed, there may remain onto the backplate 4 some portion 6 of the micro-implants as shown in Figure 2C, which is thus lost.

Furthermore, the micro-implants may not remain inserted into the keratinous materials due to skin motions and the capability to recover its initial shape after pressure on the device. A counter pressure occurs expelling the backplate away thus withdrawing the micro-implants out of the skin. Backplate's rigidity is a reason for micro-implants being rejected during skin motion. The part of the micro-implants which is not inserted cannot dissolve or disintegrate in the keratinous materials and is lost. The quantity of material delivered into skin may thus be relatively low and the efficacy of the treatment weaker than expected.

### Summary of the invention

There is a need for improving further devices for injecting a material into keratinous materials and overcoming at least some of the drawbacks of the prior art devices. It would be beneficial to improve delivery and to reduce wastes.

Exemplary embodiments of the invention relate to an injection system for injecting at least one material into human keratinous materials, comprising:
∘ At least one treatment unit defining at least one cavity and at least one micro-implant made of said at least one material received in said cavity and having at least one portion having a same shape as the corresponding cavity;
∘ an injection device configured for applying a pressure to the at least one micro-implant to expel the latter out of the unit into the keratinous materials.

Thanks to the invention, a core of the unit defining at least partially the at least one cavity may be used to mold the micro-implants and the core may be used as a package for protecting and holding the micro-implants until use. This may render the packaging of the micro-implants less expensive and/or cumbersome, as there may be no further need of a hard packaging. The micro-implants may be better protected from pressure, humidity or other environment factors by the unit until use. The cost of the packaging may be reduced.

Furthermore, the invention makes it easier to inject the entire micro-implants in the body, with no loss of material. Wastes are reduced and delivery is increased.

The absence of a conventional backplate helps penetration of the micro-implants into the stratum corneum, dermis or epidermis and induce a better delivery of the material to be injected, even in skin regions of high suppleness.

The invention enables to use micro-implants having shapes that could not be unmolded, such as shapes with cutbacks. The invention also enables shapes that would brake during a conventional de-molding process. The invention may enable to make a micro-implant using a combination of different materials that would render the micro-implant difficult to unmold.

The invention also helps to associate micro-implants of different materials in a same treatment unit.

The unit may be pressed against the body before injection. The unit may have for this purpose an application surface for contacting the keratinous materials when the at least one micro-implant is expelled out of the unit.

### Unit

The unit may define a plurality of cavities, a plurality of micro-implants being received each in a corresponding cavity of the unit and having each at least one portion of a same shape as the corresponding cavity. The unit may comprise an array of cavities. This array may comprise cavities regularly or randomly spaced along two directions that are perpendicular to each other. The shape of a cavity may correspond exactly to the shape of an entire micro-implant. The height of the cavity may thus correspond exactly to the height of the micro-implant. All the longitudinal axes of the cavities may be parallel to each other. In a variant, the longitudinal axes of the cavities may not be parallel. Cavities with non-parallel axes may allow the core to better conform to some portion in relief of the body.

The core is preferably polymeric, e.g. elastomeric, the core comprising for example a silicone elastomer. In such a case, the core may deform in reversible manner during injection of the micro-implants. The core may be squeezed during the injection. The core may also be teared or pierced to some extent by the micro-implants during injection. The core may be deformed during injection so that the longitudinal axes of at least two cavities take an orientation one relative to the other.

In a variant, the core is non-elastomeric. The core may comprise at least one material selected among PMMA, PDMS or other polymers. In such case, the core may be perforated and/or broken by the micro-implants during injection.

The core may be soluble in a liquid, for example water or another solvent or composition. The core while dissolving may be partially injected with the micro-implants. The solubility of the core may be useful to ease extraction of the micro-implants from the core, as it may fragilize the core for example.

The core is preferably biocompatible. The core is preferably bioresorbable.

The core may be permeable to liquids or gas.

When gas is used to expel the micro-implants, the injection system may be configured in such a manner that some gas penetrates in the skin. Having the core permeable to gas may aid the gas to penetrate the skin. The gas that has penetrated the skin may contribute to expand locally the skin, which may be beneficial in some applications where one seeks to bring volume to the skin such as wrinkle reduction.

The core may be squeezable thanks to the use of an elastomeric material or thanks to the presence of voids and/or micro spaces or channels that can collapse under the pressure exerted to expel the micro-implants. The core may have a solid structure or may be made by shaping a sheet to form the cavities. The core may have a composite structure with a solid body defining hollows and an internal liner present at least in said hollows to define the boundaries of cavities in which the micro-implants are located. This may enable the use of a body of low mechanical strength and high porosity, while the presence of the liner allows to mold the micro-implants with a precise shape and surface state.

The unit may comprise at least one puncturable region through which the at least one micro-implant is expelled. The presence of a puncturable region provides a protective barrier at the distal end of the micro-implants and may help preserve sterile conditions of the micro-implants.

The unit may comprise at least one puncturable sealing membrane through which the at least one micro-implant is expelled. The sealing membrane may comprise a metal, preferably aluminum and/or a polymer, for example polypropylene The sealing membrane may be removed or not before injection. When the sealing membrane is not removed, it is preferably biocompatible and/or bioresorbable.

The unit may comprise a protective cover fixed onto the core on the proximal side of at least one micro-implant. The protective cover may be detachable from the core prior to the use of the system.

The unit may comprise between 1 and 5000 micro-implants per cm². The area with micro-implants may be of outline circumscribed to a circle of diameter ranging from 3 mm to 80 mm, better from 5 mm to 50 mm.

The spacing within the array of two adjacent micro-implants may range from 50µm to 50 mm in both *x* and *y* directions of the plane.

The thickness of the unit may range for example from 25 microns to 30 mm, preferably from 25 microns to 10 mm, for example about 3mm.

A micro-implant may have a largest transverse dimension at its base no greater than 1500 microns, for example a largest transverse dimension ranging from 1 to 1500 microns. The section of a micro-implant is preferably circular, in which case the largest diameter of a micro-implant may range from 1 to 1000 microns, preferably between 200 and 400 microns.

A length of the micro-implant may range from 100 to 1500 microns, better from 200 to 800 microns, even better from 400 to 600 microns.

The micro-implant may comprise a proximal cylindrical portion of constant section and a distal portion having a section that narrows toward the tip of the micro-implant, for example a conical distal portion. The proximal and distal portions of a micro-implant may be of substantially same length, for example lₚ * 0,8 < l_{d} < lₚ*1.2, where lₚ is the length of the proximal portion and l_{d} is the length of the distal portion. The length of the proximal portion may range from 200 to 400 micron and the length of the distal portion may range from 200 to 400 microns also. Other ranges are possible, of course.

The micro-implant may also have a truncated distal portion, or a chamfered distal portion, or a beveled distal portion.

The micro-implant may be at least partially porous to gas or liquid.

The core may comprise cavities each containing a column made of a plurality of micro-implants superimposed axially. The number of micro-implants in a column may be reduced by one micro-implant each time an injection takes place. The force for pushing the micro-implant situated at the distal end of the column into the skin may be exerted on the micro-implant situated at the opposite end of the column. When the unit contains at least one column made of a plurality of micro-implants, all the micro-implants of the column may be identical or different. For example, the concentration of one compound may vary gradually along the micro-implants of the column, or two consecutive micro-implants may comprise different compounds.

The unit may be discarded after first use or after all micro-implants have been expelled therefrom. The unit can be re-usable to generate new micro implants. The core, when empty, may be used for molding new micro-implants. The material of the core may be re-shaped to form new mold cavities.

All the micro-implants may be made of a same material. In a variant, some of the micro-implants are made of a first material and some others are made of at least one second material different from the first one.

The at least one micro-implant may be made of different materials; for example, a proximal portion of the micro-implant is made in a first material and a distal portion of the micro-implant is made of a second material different from the first one. The micro-implant may comprise at least two portions of different hardness. The micro-implant may comprise a distal portion of a greater hardness than that of a proximal portion. Having a distal portion of greater hardness may help perforate the skin.

The at least one micro-implant may comprise at least one anchoring relief for anchoring the micro-implant into the keratinous materials. The at least one micro-implant may comprise at least one of a serrated or harpooning head. The invention allows formation of cutbacks in the micro-implants.

The at least one micro-implant may comprise at least one material selected among implantable substances such an implantable class of hyaluronic acid (HA), PVP, PEG or xylitol. The material of the micro-implants may be pure HA or be based on HA and xylitol.

The at least one micro-implant may carry actives that may be cosmetic actives, or drugs or vaccines. The micro-implant may be made of a material that is resorbable, preferably water-dissolvable or soluble in any body fluid.

The at least one micro-implant may comprise at least two materials in dry state that are selected so as to react when brought into contact with a body fluid. The reaction may cause a gas to be generated. This gas may cause a local expansion of the skin, which may be useful for wrinkle reduction.

The material of the micro-implants may be of high swell ability and high viscoelasticity.

The unit may have a disk shape. The unit may have other shape, such as a pellet shape with no circular outline, for example a polygonal outline, or a band or ribbon shape.

The unit may comprise at least two micro-implants of different materials, volumes, lengths and/or shapes. Micro-implants of different materials, volumes, lengths and/or shapes may be situated in respective distinct areas of the unit or may be distributed uniformly within the array.

A rear face of the or each of the micro-implant may be free and deprived of attachment to a backing layer.

The unit may comprise a liquid in contact with the at least one micro-implant. This liquid may be in contact with the micro-implant(s) in the packaging of the unit, or the liquid may be in contact just before or at the time of use, as a result of an action from the user. For example, the liquid is encapsulated and released when the micro-implants are expelled or just prior to the ejection.

As mentioned above, a micro-implant may comprise at least one cutback preventing it from being unmolded. This may help the micro-implant to resist backpressure exerted by the tissue in which it is injected. This may help reduce loss a material.

The tips of the micro-implants may be situated in a same plane. A face of the unit intended to contact the skin may be planar. In a variant, the tips of the micro-implants are not situated in a same plane, and the face of the unit intended to contact the skin may not be planar. For example, this face is having a shape that matches the shape of the skin in the area to be treated. For example, the face is having a shape convex toward the outside, and the tips are situated along a surface that is parallel to this face. Since the micro-implants need not be unmolded, the non-planar shape does not raise unmolding issues.

A further object of the invention is a treatment unit suitable for use in a system as defined above, comprising:
- a core defining at least partially at least one cavity,
- at least one micro-implant made of said at least one material and received in said cavity of the core and having at least one portion having a same shape as the cavity.

Such unit may be provided to the user in a packaging that is to be opened or not before use.

Such a packaging may be a bag and do not need to be rigid, as the core provided some protection to the micro-implants. Such packaging can be a tube with one or two caps at each end.

### Injection device

The injection device may comprise an impacting member configured for contacting a proximal end of the at least one micro-implant to expel it out of the unit.

The impacting member may be driven by any appropriate means such as for example an electromechanical actuator, a spring, a pneumatic or hydraulic device, a pyrotechnic device, or a pressure exerted manually on a surface and transferred to the micro-implants.

Manual pressure may be exerted directly on the unit or impacting member to push the micro-implants into the skin.

The impacting member may have a contact surface configured for contacting simultaneously the proximal end of a plurality of micro-implants. This surface may be flat or have any other appropriate shape, for example if the face of the unit that contacts the skin is not planar but convex.

The unit may comprise a squeezable core to allow the impacting member to push the micro-implants along a distance long enough for them to leave the core. The impacting member may have a plurality of individual projections each for contacting a respective proximal end of a micro-implant. The presence of projections (also called "counter-needles"), which may be rods, may enable to use a rigid core as the core does not need to collapse for the micro-implants to be pushed out of the core. The projections may enter the cavities to push the micro-implant out thereof.

The impacting member may impact a plurality of micro-implants simultaneously or in a sequence. In the latter case, the impacting member may oscillate between an impacting position and a raised position and move, between two consecutive impacts, to be positioned when in the raised position so as to be in line with the next micro-implant to impact.

The system may comprise at least one chamber for receiving the unit.

The system may be configured for selective expelling of the micro-implants. The injection device and the unit may be movable relative to each other for selection of the at least one micro-implant to expel. For example, the injection device and the unit are rotatable one relative to the other for selection of the at least one micro-implant to expel.

The system may comprise an energy generator, e.g. a motor or a spring, for displacing the injection device relative to the unit or vice versa.

The system may be configured for bringing into contact a liquid with the at least one micro-implant. The bringing into contact may occur prior to expelling the at least one micro-implant.

The bringing into contact may occur after the at least one micro-implant has been injected into the keratinous materials.

The system may comprise a reservoir for storing said liquid.

The liquid and the material of the micro-implants may be selected so that the liquid dissolves the material or react to generate another material. The liquid may also cause the material of the micro-implant to swell.

The system may be configured for extemporaneously bringing said liquid into contact with the micro-implants.

The system may comprise an image sensor and a processor for automated detection of a target zone of the keratinous material and for signaling a user when the system is properly positioned relative to the target zone prior to expelling the at least one micro-implant and/or for automated triggering of the injection of the at least one micro-implant when the system is properly positioned relative to the target zone. The image sensor may be an optical sensor such as a camera or a non-optical sensor such as a capacitive sensor.

The systems may be connected by a wireless connection or by a cable to a terminal such as a smartphone or a diagnosis device. The wireless connection may be Wi-Fi or Bluetooth.

The target zone may comprise a wrinkle and/or any skin depression or other skin default, such as for example achromia.

When the image sensor is off-centered relative to the micro-implants, the system is configured for detecting the displacement of the injection system along the skin to compensate this off-centering before triggering the injection. This displacement may be detected optically.

The system may comprise an electromechanical actuator for vibrating the unit and/or the injection device. This may help to create a massaging action against pain and/or improving the diffusion of the material within the stratum corneum, epidermis or dermis.

The system may comprise a cold source for lowering the temperature of the unit and/or of the keratinous materials prior to, during or after the injection. This may help reduce inflammatory side effects, pain and/or make the dermis or epidermis firmer.

The system may comprise a heat source for increasing the temperature of the unit and/or the keratinous materials prior to, during or after the injection. This may lower the rigidity of the material of the core and help micro-implants to be expelled therefrom.

The system may comprise a light source for projecting light onto a target zone where the at least one micro-implant is to be injected. This may help the user to position the system in the area to be treated and/or facilitate detection of wrinkles or other element to treat with the system.

The system may comprise at least one electrode for subjecting a target zone where the at least one micro-implant is to be injected to a microcurrent. The microcurrent may help the material of the micro-implant to penetrate deeper in the tissue, for example by pushing some actives into the skin by iontophoresis. The microcurrent may also generative actives or enhance some inner reactions.

The injection device may be removed right after the injection or left in place for a predetermined amount of time.

The system may comprise a storage compartment for storing a plurality of the treatment units. Such a storage compartment may be a cartridge in which a set of units are present. The user may insert the cartridge into the injection device and replace it by a new one after all units of the cartridge have been used.

The injection device may be configured for holding more than one cartridge, which may contain units with micro-implants of different volumes, materials and/or length or shapes.

The system may comprise a feeding mechanism configured for automatic replacement of a used unit present by a fresh unit coming from the storage compartment.

The system may be configured for automatic identification of a unit. The injection device may operate under different conditions depending the unit that has been identified.

The system may allow its operation in any direction such as for example head up or down.

### Micro-implant

A further object of the invention is a micro-implant having at least one cutback preventing it from being unmolded, for example an arrow shape.

A further object is a micro-implant of composite structure, having at least two different materials.

A further object is a column of micro-implants, present in a cavity of the unit.

Any of these micro-implants may present any of the features detailed above.

### Method of manufacturing

A further object of the invention is a method of manufacturing a unit used in a system as defined above, comprising molding the at least one micro-implant in the corresponding cavity of the unit.

The method may comprise injecting into said cavity a pourable precursor of the material of the micro-implant and having said precursor harden in said cavity. The material of the micro-implant may dry in the cavity.

The material of the micro-implant is selected to be capable of creating openings in the stratum corneum, epidermis or dermis. Preferably, the micro-implants do not fracture when force is exerted on the unit to expel the micro-implant into the skin.

The method may comprise sterilizing the unit, for example using gamma or beta ray, autoclave or chemical sterilization.

The core serving to mold the material of the micro-implants may receive some mechanical treatment. For example, holes of slits are made in the core to facilitate the deformation thereof when the micro-implants are expelled. These holes or slits made be made with a laser. The core may receive a surface treatment to lower or enhance its hydrophilicity or hydrophobicity for example.

The method may comprise molding a column of units in a cavity of the core. The column may comprise frangible areas facilitating separation of the micro-implants. The micro-implants of the column may be molded one after the other in the core. Two successive micro-implants of the column may be separated by a material facilitating separation of one micro-implant from the successive one in the column during the injection process.

The method may comprise molding the column in an elongated core that is cut to form the units. The cut line may be perpendicular to the elongation axis of the core or in a variant may be oblique to form micro-implants with beveled ends.

### Non-therapeutic method

A further object of the invention is a non-therapeutic method for treating keratinous materials, comprising injecting at least one micro-implant into the keratinous materials using the system as defined above. Preferably, the at least one micro-implant is integrally expelled from the unit during the injection.

The method may be performed for treating wrinkles.

The core, if elastically deformable and not damaged by the injection, may be re-used in the manufacture of another unit. Otherwise, it may be discarded.

Preferably, the core does not penetrate in the skin when the micro-implants are expelled. However, in a variant, some portion of the core may penetrate in the skin together with the micro-implant. The material of the core is, in the latter case, preferably bio-compatible and/or bioresorbable, and may be selected to dissolve or disintegrate in the skin. The core may be water soluble.

### Brief description of the drawings

The invention will be better understood in view of the following description of non-limitative embodiments and in view of the appended drawings in which:
Fig. 1 is a schematic view of a mold used for the manufacture of micro-implants according to the prior art,
Figs. 2A to 2C illustrate the unmolding and the use of the micro-implants according to the prior art,
Fig. 3 is a partial and schematic view in longitudinal section of an injection system according to an exemplary embodiment of the invention,
Fig. 4 is a perspective view of the device of Fig.3,
Fig. 5 shows in isolation a micro-implant,
Fig. 6 shows a treatment unit comprising micro-implants according to a variant embodiment,
Fig. 7A illustrates the ejection of the micro-implants out of the unit of Fig.6,
Fig. 7B illustrates the injection of the micro-implants of the unit of Fig.6 in the dermis or epidermis,
Fig. 7C shows the unit of Fig.6 after ejection of the micro-implants,
Figs. 8 and 9 show variant embodiments of treatment units,
Fig. 10 is a schematic and partial view of a variant embodiment of a device in accordance with the invention,
Figs. 11 and 12 are similar views to Fig.10 of variant embodiments,
Fig. 13 shows a set of treatment units,
Fig. 14 shows a variant embodiment of a unit,
Fig. 15 is a schematic block diagram of a variant of an injection device,
Fig. 16 illustrates a composite micro-implant,
Fig. 17 shows in elevation a variant embodiment of a micro-implant,
Fig. 18 shows a variant of unit,
Fig. 19 shows another variant of unit,
Fig. 20 is a schematic representation of a variant of an injection device, and
Fig. 21 shows a unit comprising a column of micro-implants.

### Detailed description of the drawings

The injecting system 10 shown in Figs.3 and 4 comprises a treatment unit 20 and an injection device 30 for acting on the unit 20.

The unit 20 comprises a core 21 and micro-implants 40 housed therein.

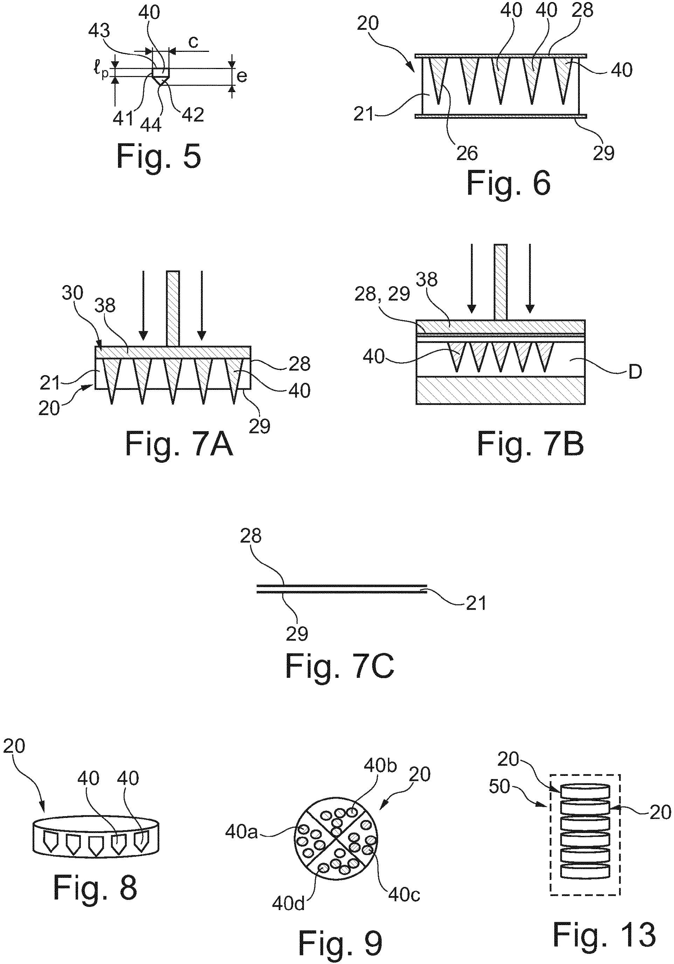

One micro-implant 40 is shown in Fig.5. It may comprise as shown a cylindrical portion 41 of length *lₚ* and diameter c and a conical head 42. The overall length *e* of the micro-implant may range from 25 µm to 2000 µm. The diameter c may range from 100 microns to 3mm. The length of the proximal portion *lₚ* may range from 50µm to 50mm. The diameter c is for example equal to 350 microns. The length e is for example equal to 500 microns. The length *lₚ* is for example equal to 250 microns.

The micro-implant 40 is to be injected in its entirety in the stratum corneum, epidermis or dermis and is made of one or more materials selected depending the desired action.

For example, the micro-implants 40 are made of hyaluronic acid or derivatives thereof.

The core 21 serves as a mold for the shaping of the micro-implants 40 and serves to protect them until use.

Various materials may be used for the core 21 depending the way the micro-implants 40 are forced out of it.

In the embodiment of Figs.3 and 4, the material of the core 21 is selected to allow the micro-implants 40 to be extracted when the impacting member of the injecting device 30 applies on their upper end 43 a force directed toward their tip 44.

This force is exerted in this embodiment by a corresponding rod 31 held by a plate 32 that moves in a cylindrical guide 33. The plate 32 may be attached by its face opposite the rods 31 to a stem 34 that is connected to a driving mechanism (not shown) configured for moving the stem forward when the micro-implants 40 are to be forced into the skin.

The driving mechanism may comprise an electromechanical device such as an electrical motor or a spring.

The invention is not limited to a specific device for driving the rods 31 and the latter may be driven in various manners based for example on a pyrotechnic device, a pneumatic or a hydraulic device, *inter alia,* or manual force.

When the rods 31 are forced against the micro-implants 40, the latter are expelled from the unit 20 and penetrate in the dermis or epidermis. The core 21 may deform elastically to allow the micro-implants to leave their corresponding cavity within the core 21. The core 21 may comprise portions that are perforated by the micro-implants 40 or torn or otherwise damaged.

The rods 31 are preferably of a cylindrical shape of same diameter as the micro-implants 40 and are centered with respect to the micro-implants 40. This way the rods 31 can penetrate into the cavities to push the micro-implants.

Their length is preferably greater than *e* so that there is no need to compress the core 21 with the plate 32 to expel the micro-implants 40 out of it.

The end face of the rods 31 is preferably of a shape that is complementary to that of the end of the micro-implants that will be impacted by the rod.

The end face of the rods 31 may be flat and perpendicular to the longitudinal axis of the guide 33, as shown. The proximal end 43 of the micro-implants 40 preferably has a corresponding flat surface, so that the area of contact between the rod and the micro-implant is maximal. This help reduce the risk of fracturing the micro-implant when pushing it out of the mold.

To use the system depicted in Figs. 3 and 4, the user places the unit 20 in a corresponding chamber of the injection device 30, and then positions the system 10 so that the unit 20 is positioned against the zone where the micro-implants should be injected.

Then, the user triggers the driving mechanism that causes the rods 31 to hit the micro-implants and expel them out of the core 21.

Once the micro-implants are within the stratum corneum, epidermis, and/or dermis the user may withdraw the injection system 10 and proceed to the replacement of the treatment unit with a new one.

Normally, the micro-implants are expelled in their entirety in the stratum corneum, epidermis or dermis, and there should not remain any non-injected portions of the micro-implants 40 trapped within the core 21.

A variant embodiment of a treatment unit 20 will now be described with reference to Fig.6.

The unit 20 comprises in this embodiment a core 21 defining cavities 26 having the same shape as the micro-implants 40 contained therein, and protective films 28 and 29 covering respectively the proximal and distal faces of the core 21.

The bottom of the cavities 26 lie at a non-zero distance from the distal face of the core 21 so that the cavities are closed at their bottom end by the material of the core 21.

The cavities 26 are closed at their opposite end by the film 28, which contacts one end of the micro-implants 40.

The films 28 and 29 constitute a barrier that helps preserve sterile conditions of the micro-implants 40.

Films 28 and 29 may remain on the core 21 when the unit is used. In a variant, the films 28 and/or 29 are removed prior the placement of the unit in the injection device.

When the films 28 and/or 29 are present in the injection device during use thereof, the films may be perforated during the injection process.

The core 21 may be made of a material than can be compressed during the injection phase, that may be performed thanks to an injection device comprising as the impacting member a pressure plate 38 as shown in Fig. 7A.

The pressure plate 38 may have a planar surface intended to contact the film 28. The pressure is transmitted to the proximal end of the micro-implants 40. The film 29 may lie on the zone where the micro-implants 40 are to be injected. The core 21 may collapse under the pressure that is exerted by the plate 38, thus allowing the micro-implants to be pushed forward through the film 29 into the dermis or epidermis D, as shown in Fig.7B.

Once the compression has ceased, the core 21 may not restore its initial shape and thickness, as illustrated in Fig.7C.

To manufacture the treatment unit 20, the core 21 is used as a mold for the material of the micro-implant or a precursor thereof. The films 28 and 29 may be attached to the core 21 after the micro-implants 40 are formed. In a variant, the film 29 may be attached to the core 21 before the material serving to make the micro-implants is poured in the cavities of the core 21. The film 28 may be attached to the core 21 before or after the material to make the micro-implants has hardened. If the film 28 is brought before the material has hardened, it may help the film 28 to adhere to the remainder of the unit 20.

A treatment unit 20 may comprise identical micro-implants 40 or micro-implants of different size and/or materials. The injection device 30 may be configured for selective injection of one or more of these micro-implants, depending on various criteria such as for example the size and/or nature of the zone to treat and/or the nature of the treatment to perform.

In the example shown in Fig.8, the unit 20 forms a sealed package in which the micro-implants are isolated from ambient air by the material of the core and/or by a sealing membrane on the proximal side of the micro-implants.

Fig.9 shows a variant of the unit 20 comprising various sets of micro-implants 40a, 40b, 40c and 40d made of different materials and/or shapes. Each set may be present in a specific region of the unit, for example a specific angular sector as shown.

The unit may comprise micro-implants of various heights and/or sizes.

The injection device may comprise a memory in which characteristics and locations of each micro-implant 40 of the unit 20 is stored. This information may be accessed based on an identifier of the unit. For example, the unit and or a packaging of the unit bears a barcode which is read by the injection system. Based on the read information, and/or on possible extra information inputted by the user on an HMI such as a keyboard or tactile screen for example, the injection system determines automatically which micro-implants should be expelled and injected in the skin.

The injection system may select automatically the micro-implants needed for injection based for example on the dose of each material to inject.

The injection device may comprise individual rods that may be controlled independently of the others. Rods can be of different length, diameter and shape. They can move up and down. There may be as many of these rods as there are micro-implants. The injection device may be configured to memorize the position of the micro-implants that have already been expelled out of the unit. In this way, the injection device can determine which rods should be actuated to expel the remaining micro-implants after each use.

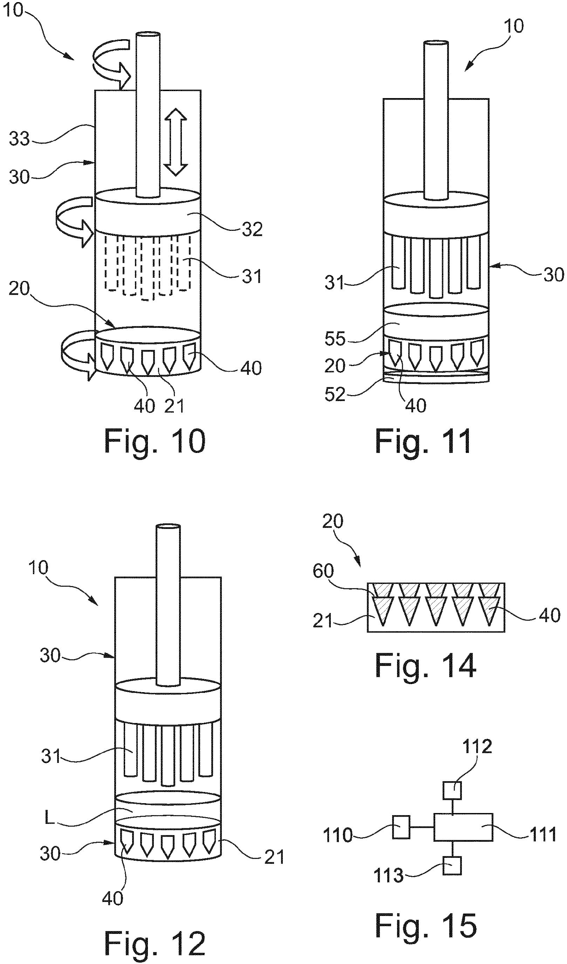

The injection device may also contain less rods than there are micro-implants and the injection system is configured for allowing a displacement of the unit relative to the injection device once all the micro-implants of a given area of the unit have been expelled. This displacement may be a rotation of the unit 20 and/or of the rods 31 and plate 32, as shown in Fig.10.

The system may also comprise one or more actuating rods that are carried by a carriage that is mobile relative to the unit in the *x* and *y* directions.

The injection system 10 may comprise at least one pressure sensor and be configured to allow injection only when a threshold pressure is exceeded on the skin. This helps reduce the suppleness of the skin and improve the penetration of the micro-implants in the skin

Fig.11 shows an injection system in which the injection device 30 is equipped with a sensor 52 represented schematically, which may be a pressure sensor. This sensor provides an indication of the pressure of the injection system 10 against the skin. The injection device 30 may comprise a processor controlling the driving mechanism used for moving the rods 31 or other pressure means serving to expel the micro-implants 40. This processor receives the signal from the pressure sensor and can trigger the injection only once a given pressure is reached.

The presence of a pressure sensor in the injection device may help generate the right pressure for micro-implant insertion according to the skin mechanical properties.

As illustrated in Fig.11, the injection system 10 may also comprise an identification sensor 55 to identify the unit 20 that is used. Based on this identification, the injection device may operate according to a specific program, for example.

Identification may be performed thanks to the presence on the unit of a specific mark, such as a barcode as mentioned above.

The core 21 may be made of a material that is soluble in a specific solvent, such as water for example. The unit 20 may be put into contact with this solvent prior to the micro-implants being expelled. The solvent may modify the rigidity of the core 21 and facilitate the deformation of the core that is necessary to allow the micro-implants to leave the core.

The injection may also take place in presence of a liquid selected to increase the solubility of the material of the micro-implants into the dermis or epidermis or other medium into which the micro-implants are injected.

This liquid L may be present above the unit 20 as shown in Fig.12. The injection device 30 may first drive the micro-implants out of the core 21 and then force some liquid to follow the micro-implants into the dermis or epidermis.

A plurality of units 20 may be packaged in a same cartridge 50, as shown in Fig.13. Such a cartridge may be tube-like.

The injection device 30 may then be configured to extract from the cartridge one unit 20 at a time. When a unit has been used, it is ejected from the system and a new unit is withdrawn from the cartridge.

The micro-implants 40 may be given various shapes, including shapes with undercuts that would prevent the micro-implants from being unmolded from the core 21.

As an example, Fig.14 shows a treatment unit 20 comprising micro-implants 40 having an arrow shape. Such a shape allows better anchoring of the micro-implants in the tissue in which it has been injected. The micro-implants comprise at least one cutback 60 preventing them from unmolding.

The injection device may be provided with a target sensor that serves to recognize a given area in order to treat it automatically. For example, the target sensor detects a wrinkle and the injection device is configured to inject the micro-implants only in the wrinkle area.

The injection device may comprise as illustrated in Fig. 15 an image sensor 110 and a processor 111 for automated detection of a target zone of the keratinous material and for signaling a user when the system is properly positioned relative to the target zone prior to expelling the at least one micro-implant and/or for automated triggering of the injection of the at least one micro-implant when the system is properly positioned relative to the target zone.

The injection device may comprise an interface 112 and an actuator 113 for actuating an impacting member to expel the micro-implants. The interface 112 may comprise a tactile screen and/or some buttons to select operating parameters.

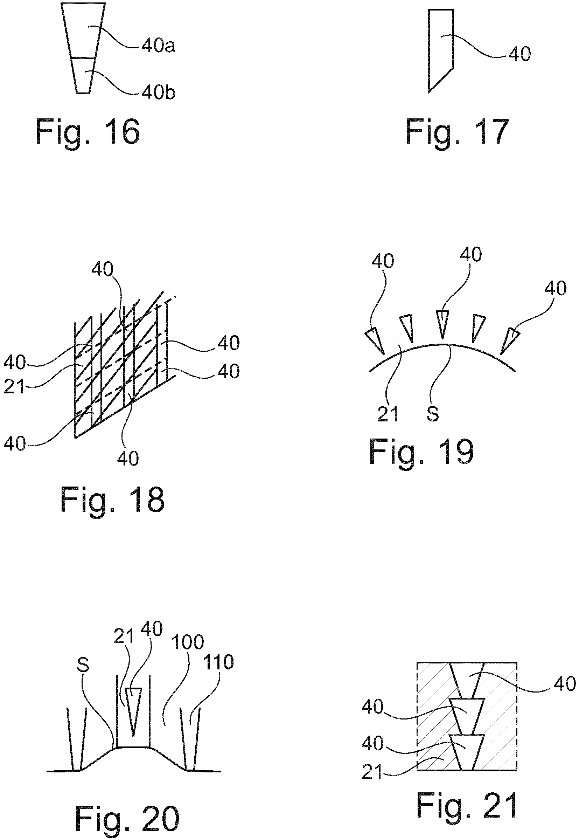

Fig. 16 illustrates the possibility for the micro-implants 40 to be of composite nature, with for example a distal portion 40b made of a material with higher hardness than the proximal portion 40a.

The distal end of the micro-implant may have various shapes. It is not necessarily conical. It may be flat, round or beveled, as shown in Fig. 17.

The core 21 may have an elongated shape and the units may be formed by cutting the elongated core in successive portions, as illustrated in Fig.19. By varying the spacing between the cutting lines, one may vary the length of the micro-implants 40. The cutting line may be oblique relative to the longitudinal axis of the core 21 so as to form beveled distal ends for the micro-implants.

The core 21 may be flexible and deformed to take the shape of the body portion S against which the unit is applied prior to injection of the micro-implants, as shown in Fig. 19.

In the variant embodiment shown in Fig.20; the injection device comprises an enclosure 110 that has an opening that is configured for being placed on the skin. A vacuum pump creates a vacuum in the space 100 inside the enclosure which causes the skin S to deform towards the unit. By varying the intensity of the vacuum, one may cause the skin to deform more or less towards the unit. In this way, the distance between the micro-implants and the skin may be varied and with it the depth of the penetration of the micro-implants into the skin. The vacuum may also help to homogenize skin properties prior to the injection.

In another embodiment shown in Fig. 21, micro-implants are superimposed axially in a column and are delivered to the skin one after the other, in a succession. The micro-implant situated at the bottom of the column is forced into the skin thanks to pressure applied on the micro-implant situated at the top. The micro-implants may be identical or made of different materials or have a varying concentration of an active ingredient. For example, the concentration varies from bottom to top, to take into account the need to vary the power of a treatment in time. The first injection delivers micro-implants with smaller concentration, and the concentration increases progressively with the following injections. In a variant; the first injection has the strongest concentration, and then the concentration decreases with the following injections.

## Claims

1. An injection system (10) for injecting at least one material into human keratinous materials, comprising:
∘ At least one treatment unit (20) defining at least one cavity (26) and at least one micro-implant (40) made of said at least one material received in said cavity of the unit and having at least one portion having a same shape as the cavity;
∘ an injection device (30) configured for applying a pressure to the at least one micro-implant (40) to expel the latter out of the unit (20) into the keratinous materials.

2. The system of claim 1, the unit (20) having an application surface for contacting the keratinous materials when the at least one micro-implant is expelled out of the unit.

3. The system of claim 1 or 2, the unit defining a plurality of cavities (26), a plurality of micro-implants being received each in a corresponding cavity of the unit and having each at least one portion of a same shape as the corresponding cavity.

4. The system of any one of claims 1 to 3, the unit comprising a core (21) defining at least partially the at least one cavity (26) and being polymeric.

5. The system of any one of the preceding claims, the unit (20) comprising at least one puncturable region through which the at least one micro-implant is expelled.

6. The system of any one of the preceding claims, the unit comprising at least one puncturable membrane (29) fixed onto a core (21) defining at least partially the at least one cavity and through which the at least one micro-implant is expelled.

7. The system of any one of the preceding claims, the unit comprising a core (21) defining at least partially the at least one cavity and the unit comprising a protective cover fixed onto the core on the proximal side of at least one micro-implant.

8. The system of any one of the preceding claims, the injection device comprising at least one impacting member (31; 38) configured for contacting a proximal end of the at least one micro-implant (40) to expel it out of the unit.

9. The system of any one of the preceding claims, the unit comprising a squeezable core (21) defining at least partially the at least one cavity (26).

10. The system of any one of claims 1 to 9, some of the micro-implants (40a) being made of a first material and some others (40b, 40c, 40d) being made of at least one second material different from the first one.

11. The system of any one of claims 1 to 10, comprising a reservoir for storing a liquid, the liquid and the material of the micro-implants being selected so that the liquid dissolves the material or react to generate another material.

12. The system of any one of the preceding claims, comprising an image sensor and a processor for automated detection of a target zone of the keratinous material, such as a skin default, and for signaling a user when the system is properly positioned relative to the target zone prior to expelling the at least one micro-implant and/or for automated triggering of the injection of the at least one micro-implant when the system is properly positioned relative to the target zone.

13. A unit (20) suitable for use in a system as defined in any one of the preceding claims, comprising:
- a core (21) defining at least partially at least one cavity (26),
- at least one micro-implant (40) made of said at least one material and received in a corresponding cavity (26) of the core (21) and having at least one portion having a same shape as the corresponding cavity.

14. A method of manufacturing a unit used in a system as defined in any one of claims 1 to 12 or as defined in claim 13, comprising molding the at least one micro-implant (40) in the corresponding cavity (26) of the unit (20).

15. A non-therapeutic method for treating keratinous materials, comprising injecting at least one micro-implant into the keratinous materials using the system as defined in any one of claims 1 to 12.
